# FASCICULE DE BREVET EUROPEEN

(11) **EP 2 822 980 B1**
(45) Date de publication et mention de la délivrance du brevet: **20.07.2016**
(21) Numéro de dépôt: 13710475.8
(22) Date de dépôt: 18.02.2013
(51) Int. Cl.: C08F 220/06, C08F 230/08, C08F 283/12, A61K 8/84, A61K 47/34, A61Q 1/00, A61Q 5/00, A61Q 19/00

(54) **NOUVEAU POLYMÈRE D'ACRYLATE DE SILICONE ET D'ACIDE ACRYLIQUE ÉPAISSISSANT ET RÉDUISANT LE CARACTÈRE COLLANT DES FORMULES COSMÉTIQUES À BASE DE GLYCÉRINE**
NEUARTIGES SILIKONACRYLAT UND ACRYLSÄUREVERDICKENDES POLYMER ZUR VERRINGERUNG DER KLEBRIGKEIT VON KOSMETISCHEN ZUSAMMENSETZUNGEN AUF GLYCERINBASIS
NOVEL SILICONE ACRYLATE AND ACRYLIC ACID THICKENING POLYMER REDUCING THE STICKINESS OF GLYCERINE-BASED COSMETIC FORMULAS

(30) Priorité: 08.03.2012 FR 1252106
(43) Date de publication de la demande: 14.01.2015
(73) Titulaire: Societe D'Exploitation De Produits Pour Les Industries Chimiques Seppic, 75007 Paris (FR)
(72) Inventeur: BRAUN, Olivier, 42170 St Just St Rambert (FR); MALLO, Paul, F-78110 Le Vésinet (FR)
(74) Mandataire: Conan, Philippe Claude
(86) Numéro de dépôt international: PCT/FR2013/050327
(87) Numéro de publication internationale: WO 2013/132169

(56) Documents cités:
- EP-A1- 1 266 914
- EP-A1- 1 750 157
- EP-A2- 1 486 516
- WO-A1-01/32727

## Description

L'invention a pour objet de nouveaux agents épaississants ainsi que leur utilisation en cosmétique et en pharmacie.

Il est bien connu d'épaissir des phases aqueuses destinées à des applications cosmétiques, dermo-pharmaceutiques ou pharmaceutiques, en y introduisant des polymères hydrophiles synthétiques ou naturels. Les polymères naturels tels que les gommes de xanthane ou de guar sont assez largement utilisés mais présentent les inconvénients classiques des produits naturels (qualité et prix fluctuants).
C'est pourquoi les polymères épaississants synthétiques sont largement utilisés dans l'industrie cosmétique, dermo-pharmaceutique ou pharmaceutique. Des épaississants fonctionnant sur une large gamme de pH et ayant l'avantage d'être particulièrement bien tolérés ont été déjà proposés par plusieurs compagnies dont la demanderesse.

On peut notamment citer les épaississants synthétiques décrits dans les brevets US 6,197,287, US 6,136,305, US 6,346,239 ou encore EP 0 503,853, ou encore US 5,004,598.

Ces polymères se présentent sous forme de latex inverse ou de poudre. Ils sont essentiellement destinés à épaissir des phases aqueuses contenant les différents constituants classiques que l'on peut trouver dans des formulations topiques de l'industrie cosmétique, dermo-pharmaceutique ou pharmaceutique. On citera notamment des huiles, des tensioactifs (non ioniques ou anioniques) encore appelés émulsionnants, des sels minéraux, des acides faibles.

Or certaines formulations, plus particulièrement celles destinées au soin de la peau, contiennent aussi des quantités relativement importantes de glycérine (ou glycérol), typiquement entre 5% et 10% massique, pour augmenter leur potentiel hydratant. Mais comme la présence de glycérine en leur sein augmente aussi considérablement leur effet collant, les préparateurs y ajoutent des huiles de silicone pour limiter ou annihiler cet effet collant.

Cependant l'addition d'huiles de silicone complexifie la préparation de ces formules. De plus, la présence d'huiles de silicone dans des formules, qui sont destinées à être en contact direct avec la peau, est mal perçue par le consommateur final.

L'industrie cosmétique essaye donc d'en limiter l'utilisation.

Aujourd'hui aucun des polymères épaississants présents sur le marché ne donne satisfaction de ce point de vue.

Les inventeurs ont donc cherché à développer de nouveaux polymères épaississants qui soient efficaces sur une large gamme de pH, et qui soient capables de réduire ou d'annihiler l'effet collant induit par la présence de glycérine, sans qu'il soit nécessaire d'ajouter un tiers composés comme les dérivés siliconés. Ils ont trouvé que des poudres de polymères issus de la polymérisation précipitante de monomères siliconés et de monomère possédant une fonction acide faible, résolvaient ces problèmes.

C'est pourquoi selon un premier aspect, l'invention a pour objet un polyélectrolyte anionique linéaire, branché ou réticulé, issu de la polymérisation pour 100% massique :
a) d'une proportion massique supérieure ou égale à 80% et inférieure ou égale à 99,5% d'unités monomériques issues d'un monomère comportant une fonction acide faible ;
b) d'une proportion massique supérieure ou égale à 0,5% et inférieure ou égale à 20%,
   - Soit d'un monomère de formule (Ia) :

      R-(CH₂)₃-Si(CH₃)₂-[O-Si(CH₃)₂-]ₙO-Si(CH₃)₂-(CH₂)₃-R (la),

      dans laquelle :
      R représente le radical monovalent :

         -(O-CH₂-CH₂-)ₓ[O-CH₂-CH(CH₃)-]_{y}O-C(=O)-CH=CH₂,

         dans lequel x et y représentent, indépendant l'un de l'autre, un nombre entier supérieur ou égal à 0 et inférieur ou égal à 40, étant entendu que la somme x + y est supérieure à 0 et inférieure à 50 ; et
      n représente un nombre entier supérieur ou égal à 0 et inférieur ou égal à 45, ledit monomère de formule (la) ayant un poids moléculaire supérieur ou égal à 1.500 et inférieur ou égal à 7.000 ;
   - Soit d'un monomère de formule (Ib) :

      Si(CH₃)₃-[O-Si(CH₃)₂-]ₘ[O-Si(CH₃)[(CH₂)₃-R]-]ₚO-Si(CH₂)₃ (Ib),

      dans laquelle :
      R représente un radical monovalent de formule :

         -(O-CH₂-CH₂-)ₓ[O-CH₂-CH(CH₃)-]_{y}O-C(=O)-CH=CH₂,

         dans lequel x et y représentent, indépendant l'un de l'autre, un nombre entier supérieur ou égal à 0 et inférieur ou égal à 40, étant entendu que la somme x + y est supérieure à 0 et inférieure à 50 ;
      m représente un nombre entier supérieur ou égal à 1 et inférieur ou égal à 7, et
      p représente indépendamment de m un nombre entier supérieur ou égal à 1 et inférieur à 20 ;
      ledit monomère de formule (Ib) ayant un poids moléculaire supérieur ou égal à 1.000 et inférieur ou égal à 4.000 ;
c) optionnellement d'une proportion massique supérieure à 0% et inférieure ou égale à 5%, d'unités monomériques issues d'au moins un monomère de formule (II) :

   R₂-C(=O)-O-[(CH₂-CH(R₄)-O]ₘ-R₃ (II),

   formule (II) dans laquelle m représente un nombre supérieur ou égal à 0 et inférieur ou égal à 50, R₂ représente un radical monovalent aliphatique insaturé comprenant de 2 à 4 atomes de carbone, R₄ représente un atome d'hydrogène, un radical méthyle ou un radical éthyle et R₃ représente un radical aliphatique hydrocarboné, linéaire ou ramifié, saturé ou insaturé, comprenant de 8 à 30 atomes de carbone, et
d) optionnellement d'une proportion massique supérieure à 0% et inférieure ou égale à 5% d'au moins un monomère de réticulation diéthylénique ou polyéthylénique.

Par polyélectrolyte branché, on désigne un polyélectrolyte non linéaire qui possède des chaînes pendantes de manière à obtenir, lorsqu'il est mis en solution dans l'eau, un fort état d'enchevêtrement conduisant à des viscosités, à bas gradient de vitesse, très importantes.

Par polyélectrolyte réticulé, on désigne un polyélectrolyte non linéaire se présentant à l'état de réseau tridimensionnel insoluble dans l'eau, mais gonflable à l'eau et conduisant donc à l'obtention d'un gel chimique.

Le polyélectrolyte obtenu par le procédé selon l'invention peut comporter des motifs réticulés et/ou des motifs branchés.

Par monomère comportant une fonction acide faible, on désigne notamment les monomères choisis parmi l'acide acrylique, l'acide méthacrylique, l'acide itaconique, l'acide maléique ou l'acide 3-méthyl 3-[(1-oxo 2-propènyl) amino] butanoïque.

Selon un aspect particulier, ledit monomère comportant une fonction acide faible, est l'acide acrylique.

Selon un autre aspect particulier, le polyélectrolyte linéaire, branché ou réticulé, est caractérisé en ce que la proportion massique en unités monomériques issues du monomère comportant une fonction acide faible, est inférieure ou égale à 98%.

Dans la formule (II) telle que définie précédemment, le radical divalent :

-[(CH₂-CH(R₄)-O]ₘ-

représente notamment :
- Soit une chaîne composée uniquement de groupes éthoxyle (R₄ = H ; n >0),
- Soit une chaîne composée uniquement de groupes propoxyle (R₄ = CH₃ n > 0),
- Soit une chaîne composée uniquement de groupes butoxyle (R₄ = C₂H₅ ; n> 0),
- Soit une chaîne composée d'au moins deux groupes différents choisis parmi les groupes éthoxyle propoxyle et/ou butoxyle.

Lorsque cette chaîne est composée de groupes différents, ils sont distribués tout au long de cette chaîne, de façon séquencée ou aléatoire.

Par radical aliphatique, hydrocarboné linéaire, saturé ou insaturé, comprenant de 8 à 30 atomes de carbone, on désigne plus particulièrement pour R₃, dans la formule (II) telle que définie précédemment :
- Soit un radical dérivé des alcools primaires linéaires tels que par exemple, ceux dérivés des alcools octylique, pélargonique, décylique, undécylique, undécénylique, laurique, tridécylique, myristylique, pentadécylique, cétylique, heptadécylique, stéarylique, oléylique, linoléylique, nonadécylique, arachidique, béhènylique, érucylique ou I-triacontanoïque. Il s'agit alors des radicaux octyle, nonyle, décyle, undécyle, 10-undécènyle, dodécyle, tridécyle, tétradécyle, pentadécyle, hexadécyle, heptadécyle, octadécyle, 9-octadécènyle, 10,12-octadécadiènyle, 13-docosènyle ou triacontanyle ;
- Soit un radical dérivé des alcools de Guerbet, qui sont des 1-alcanols ramifiés répondant à la formule générale :

   CH₃-(CH2)ₚ-CH[CH3-(CH2)ₚ₋2]-CH₂OH,

   dans laquelle p représente un nombre entier compris entre 2 et 14, tels que, par exemple, les radicaux 2-éthyl hexyle, 2-propyl heptyle, 2-butyl octyle, 2-pentyl nonyle, 2-hexyl décyle ou 2-octyl dodécyle ;
- Soit un radical dérivé des isoalcanols répondant à la formule générale :

   CH₃-CH(CH₃)-(CH₂)ₘ-CH₂OH,

   dans laquelle m représente un nombre entier compris entre 2 et 26 tels que, par exemple, les radicaux 4-méthyl pentyle, 5-méthyl hexyle, 6-méthyl heptyle, 15-méthyl pendadécyle ou 16-méthyl heptadécyle ;
- Soit les radicaux 2-hexyl octyle, 2-octyl décyle ou 2-hexyl dodécyle.

Par radical aliphatique hydrocarboné, linéaire ou ramifié, saturé ou insaturé, comprenant de 8 à 30 atomes de carbone, on désigne plus particulièrement pour R₃; dans la formule (II) telle que définie précédemment, un radical alkyle comportant de 12 à 22 atomes de carbone.

Dans la formule (II) telle que définie précédemment, m représente plus particulièrement un nombre supérieur ou égal à 0 et inférieur ou égal à 25.

Dans la formule (II) telle que définie précédemment, R₂ représente plus particulièrement le radical vinyle (CH₂=CH-) ou le radical isopropènyle [CH₂=C(CH₃)-].

Selon un aspect plus particulier de la présente invention, ledit monomère de formule (II) telle que définie précédemment, est choisi parmi :
- Le méthacrylate de béhènyle pentacosaéthoxylé, composé de formule (II) telle que définie précédemment, dans laquelle R₃ représente le radical docosanyle, R₂ représente le radical isopropènyle, R₄ représente un atome d'hydrogène et n est égal à 25;
- L'acrylate de lauryle tétraéthoxylé, composé qui correspond à la formule (II) telle que définie précédemment, dans laquelle R₃ représente le radical dodécyle, R₂ représente le radical vinyle, R₄ représente un atome d'hydrogène et n est égal à 4,
- Le méthacrylate de stéaryle eicosaéthoxylé, composé de formule (II) telle que définie précédemment, dans laquelle R₃ représente le radical stéaryle, R₂ représente le radical isopropènyle, R₄ représente un atome d'hydrogène et n est égal à 20,
- Le méthacrylate de lauryle tétraéthoxylé, composé qui correspond à la formule (I) telle que définie précédemment, dans laquelle R₃ représente le radical dodécyle, R₂ représente le radical isoprènyle, R₄ représente un atome d'hydrogène et n est égal à 4, ou
- Le méthacrylate de stéaryle, composé de formule (II) telle que définie précédemment, dans laquelle R₃ représente le radical stéaryle, R₂ représente le radical isopropènyle, R₄ représente un atome d'hydrogène et n est égal à 0.

Selon un aspect plus particulier de la présente invention, ledit monomère de formule (la) telle que définie précédemment, est le SILMER™ ACR Di-50 commercialisé par la société SILTECH, de poids moléculaire égal à 4.100 et référencé dans le "Chemical Abstracts sous le numéro de registre RN = 128754-61-0.

Selon un aspect plus particulier de la présente invention, ledit monomère de formule (la) telle que définie précédemment, est le SILMER™ ACR Di-10 commercialisé par la société SILTECH, de poids moléculaire égal à 1.100.

Selon un aspect plus particulier de la présente invention, ledit monomère de formule (Ib) telle que définie précédemment, est le SILMER™ ACR D208 commercialisé par la société SILTECH, de poids moléculaire égal à 3.000 et référencé dans le "Chemical Abstracts" sous le numéro de registre RN= 518299-28-0

Selon un autre aspect plus particulier de la présente invention, ledit monomère de formule (Ib) telle que définie précédemment, est le SILMER™ ACR D2 commercialisé par la société SILTECH, de poids moléculaire égal à 1.400 et référencé dans le "Chemical Abstracts" sous le numéro de registre RN = 158061-40-6.

Selon un aspect particulier, l'invention a plus particulièrement pour objet un polyélectrolyte tel que défini précédemment, issu de la polymérisation pour 100% massique :
a) d'une proportion massique supérieure ou égale à 85% et inférieure ou égale à 98% d'unités monomériques issues d'un monomère comportant une fonction acide faible,
b) d'une proportion massique supérieure ou égale à 1% et inférieure ou égale à 14% d'unités monomériques issues d'un monomère de formule (la) ou d'un monomère de formule (Ib) telles que définies précédemment ; et
c) d'une proportion massique supérieure ou égale à 1% et inférieure ou égale à 5% d'unités monomériques issues du composé de formule (II) telle que définie précédemment.

Selon un autre aspect particulier de la présente invention, le polyélectrolyte tel que défini ci-dessus est réticulé.

Selon ce dernier aspect, ledit au moins un monomère de réticulation diéthylénique ou polyéthylénique est choisi notamment parmi l'acide diallyloxyacétique ou un des sels comme son sel de sodium, le triallylamine, le triacrylate de triméthylol propane, le diméthacrylate d'éthylèneglycol, le diacrylate de diéthylène glycol, le diallylurée ou le méthylène bis(acrylamide) ou un mélange de plusieurs de ces composés.

Selon un aspect tout particulier de la présente invention, l'agent réticulant mis en oeuvre est le méthylène bis(acrylamide) ou le triacrylate de triméthylol propane (TMPTA).

L'agent de réticulation est alors généralement mis en oeuvre dans la proportion molaire exprimée par rapport aux monomères mis en oeuvre, de 0,005% massique à 5% massique, et plus particulièrement de 0,5% massique à 2,5% massique.

L'invention a aussi pour objet un procédé de préparation du polyélectrolyte tel que défini précédemment, caractérisé en ce qu'il comprend:
Une étape a) de préparation d'un mélange réactionnel comprenant dans les proportions molaires souhaitées et dans un solvant organique (S), le ou les monomères comportant une fonction acide faible, le monomère de formule (la) ou le monomère de formule (Ib) ; si nécessaire ou si désiré les unités monomériques issues du composé de formule (II) ; et si nécessaire ou si désiré le ou les monomères de réticulation diéthyléniques ou polyéthyléniques,
Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte.

Selon un autre aspect particulier de la présente invention, dans l'étape b) du procédé tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis(isobutyronitrile) ou encore les dérivés azoïques.

Selon un autre aspect particulier de la présente invention, dans l'étape b) du procédé tel que défini précédemment, la réaction de polymérisation est amorcée par un couple oxydoréducteur tel qu'un couple oxydoréducteur générateur d'ions hydrogénosulfite (HSO₃) comme le couple hydroperoxyde de cumène - métabisulfite de sodium (Na₂S₂O₅) ou le couple hydroperoxyde de cumène-chlorure de thionyle (SOCl₂) à une température inférieure ou égale à 20°C, si désiré accompagné d'un agent co-initiateur de polymérisation tel que par exemple l'azo-bis(isobutyronitrile), le peroxyde de dilauryle, puis conduite de manière quasi-adiabatique.

Le procédé tel que défini ci-dessus peut comprendre en outre :
une étape c) d'isolation dudit précipité obtenu à l'étape b) par séparation d'avec ledit solvant (S), puis si nécessaire ou si désiré,
une étape d) de séchage dudit précipité résultant de l'étape c).

Selon un autre aspect particulier de la présente invention, dans l'étape c) du procédé tel que défini précédemment, la séparation du précipité obtenu dudit solvant organique est effectué par filtration.

Selon un autre aspect particulier, l'invention a pour objet un procédé tel que défini ci-dessus, dans lequel ledit solvant organique (S) est choisi le toluène, le benzène, le cyclohexane, l'heptane, l'acétate d'éthyle, le trichlorométhane, le dichloro-éthane, ou un mélange de ces derniers.

Selon un aspect plus particulier, l'invention a pour objet un procédé tel que défini ci-dessus, dans lequel le solvant (S) est un mélange cyclohexane/acétate d'éthyle.

L'invention a aussi pour objet l'utilisation du polyélectrolyte anionique tel que défini précédemment, comme épaississant et/ou comme stabilisant et/ou comme émulsionnant, d'une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile-dans-eau (H/E), eau-dans-huile (E/H), huile-dans-eau-dans-huile (H/E/H) ou eau-dans-huile-dans-eau (E/H/E). La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple, consister en un agent anti-inflammatoire, un myorelaxant, un antifongique, un antibactérien ou antipelliculaire.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un antirides, un agent à visée amincissante, un agent antiradicalaire, un agent anti-acnéique, un antifongique ou antipelliculaire.

La composition topique selon l'invention comporte habituellement entre 0,1% et 10% massique et plus particulièrement de 1% à 5% massique du polyélectrolyte anionique tel que défini précédemment.

Selon un aspect particulier la composition topique telle que définie ci-dessus comprend en outre de 1% massique à 10% massique de glycérol.

Le pH de la composition topique est de préférence supérieur ou égal à 3.

La composition topique peut en outre comporter des composés classiquement compris dans ce type de compositions, par exemple des parfums, des conservateurs, des colorants, des pigments, des écrans solaires, des ingrédients actifs, des émollients ou des tensioactifs.

Le polyélectrolyte anionique selon l'invention est un substitut intéressant aux latex inverses vendus sous les noms de SEPIGEL™ 305, SEPIGEL™ 501, SIMULGEL™ EG, SIMULGEL™ EPG, SIMULGEL™ NS, SIMULGEL™ INS 100, SIMULGEL™ 600, SIMULGEL™ A, SEPIPLUS™ 265, SEPIPLUS™ 250, SEPIPL US™ 400 ou SEPIPLUS™ S par la demanderesse, car il présente aussi une bonne compatibilité avec les autres excipients utilisés pour la préparation de formulations telles que les laits, les lotions, les crèmes, les savons, les bains, les baumes, les shampooings ou les après-shampooings. Il peut aussi être mis en oeuvre avec lesdits SEPIGEL™ et/ou SIMULGEL™ et/ou SEPIPLUS™.

Il est notamment compatible avec les concentrés décrits et revendiqués dans les publications internationales WO 92/06778, WO 95/04592, WO 95/13863, WO 96/37285, WO 98/22207, WO 98/47610 ou dans FR 2 734 496, avec les agents tensioactifs décrits dans WO 93/08204.II est particulièrement compatible avec le MONTANOV™ 68, le MONTANOV™ 82, le MONTANOV™ 202, le MONTANOV™ L, le MONTANOV ™ S, le FLUIDANOV™ 20X ou l'EASYNOV™.

Il peut également être utilisé pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 19523596.

Il est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou WO 98/09611. Il est également compatible avec des polymères épaississants et/ou gélifiants comme les hydrocolloïdes d'origine végétale ou biosynthétique, par exemple la gomme xanthane, la gomme de karaya, les carraghénates, les alginates, les galactomannanes ; comme les silicates ; comme la cellulose et ses dérivés ; comme l'amidon et ses dérivés hydrophiles ; comme les polyuréthanes.

Le polyélectrolyte anionique selon l'invention permet de plus de s'affranchir de l'utilisation d'huile de silicone dans les compositions topiques comprenant du glycérol, en ce qu'elle inhibe l'effet collant induit par ce triol.

C'est pourquoi, selon un dernier aspect, l'invention a pour objet une composition topique comprenant entre 0,1 % et 10 % massique et plus particulièrement de 1 % à 5 % massique du polyélectrolyte anionique tel que défini précédemment et de 1% massique à 10% massique de glycérol et caractérisée en ce qu'elle est exempte d'huile de silicone

Les exemples suivants illustrent l'invention sans toutefois la limiter.

### Exemple 1: Préparation d'un copolymère AA/ACR-D2

On charge dans un réacteur maintenu à 25°C sous agitation et contenant 270g d'acétate d'éthyle et 230g de cyclohexane, 88,2g d'acide acrylique (AA).

Après un temps suffisant pour atteindre une bonne homogénéisation de la solution, celle-ci est désoxygénée par barbotage d'azote, puis on ajoute 7,1g de SILMER™ ACR D2 (composé de formule (Ib), identifié sous le numéro Chemical Abstract RN= 158061-40-6, de poids moléculaire égal à 1400).

On laisse le mélange réactionnel sous agitation pendant soixante minutes; puis on le chauffe jusqu'à atteindre la température de 60°C. On y ajoute alors 0,5g de peroxyde de dilauroyle. Le milieu réactionnel est ensuite de nouveau laissé sous agitation pendant environ 60 minutes puis porté à 80°C et laissé à cette température pendant cinq heures. Après refroidissement, la poudre qui s'est formée en cours de polymérisation, est filtrée et séchée pour obtenir le produit désiré, dénommé par la suite : Polyélectrolyte 1.

### Evaluation du pouvoir épaississant

Viscosité (µ) d'une dispersion aqueuse à 0,5% massique de Polyélectrolyte 1, le pH étant ajusté à 7 [Brookfield RVT, Mobile 6, Vitesse : 5 tours/minute (M6, V5)] : µ = **70.200 mPas.**

Viscosité (µ) d'une dispersion aqueuse à 1% massique de Polyélectrolyte 1, le pH étant ajusté à 7 et 1‰ massique de chlorure de sodium [Brookfield RVT, (M6, V5)] : **µ = 7.400 mPas.**

### Evaluation du caractère collant de gels aqueux contenant de la glycérine

On prépare un gel aqueux en mélangeant 1,875g du Polyélectrolyte 1, 25g de glycérine et 223,125g d'eau permutée.

A titre de base de comparaison on prépare un gel aqueux en mélangeant 4,7g de SEPIGEL™ 305, 25g de glycérine et 220,3g d'eau.

En étalant chacun des deux gels sur une surface différente du dessus de la main, on observe l'absence d'effet collant pour le gel comprenant le Polyélectrolyte 1 selon l'invention contrairement au gel selon l'état de la technique.

### Exemple 2 : Préparation d'un copolymère AA/ACR D2

On opère de la même façon qu'à l'exemple 1 mais en réduisant la quantité de SILMER™ ACR D2 mise en oeuvre à 4,4g. On obtient le Polyélectrolyte 2.

### Evaluation du pouvoir épaississant

Viscosité (µ) d'une dispersion aqueuse à 0,5% massique de Polyélectrolyte 2, le pH étant ajusté à 7 [Brookfield RVT, (M6, V5)] : **µ = 108.000 mPas.**

Viscosité (µ) d'une dispersion aqueuse à 1% massique de Polyélectrolyte 2 le pH étant ajusté à 7 et 1‰ massique de chlorure de sodium [Brookfield RVT, (M6, V5)] : **µ = 12.000 mPas.**

### Evaluation du caractère collant de gels aqueux contenant de la glycérine

On prépare un gel aqueux en mélangeant 1,875g du Polyélectrolyte 2, 25g de glycérine et 223,125g d'eau permutée.

A titre de base de comparaison on prépare un gel aqueux en mélangeant 4,7g de SEPIGEL™ 305, 25g de glycérine et 220,3g d'eau.

En étalant chacun des deux gels sur une surface différente du dessus de la main, on observe l'absence d'effet collant pour le gel comprenant le Polyélectrolyte 2 selon l'invention contrairement au gel selon l'état de la technique.

### Exemple 3 : Préparation d'un copolymère AA/ACR D2

On opère de la même façon qu'à l'exemple 1 mais en réduisant la quantité de SILMER™ ACR D2 mise en oeuvre à 2,6g. On obtient le Polyélectrolyte 3.

### Evaluation du pouvoir épaississant

Viscosité (µ) d'une dispersion aqueuse à 0,5% massique de Polyélectrolyte 3, le pH étant ajusté à 7 [Brookfield RVT, (M6, V5)] : **µ = 39.800 mPas.**

Viscosité (µ) d'une dispersion aqueuse à 1% massique de Polyélectrolyte 3 le pH étant ajusté à 7 et 1‰ massique de chlorure de sodium [Brookfield RVT, (M6, V5)] : **µ = 15.800 mPas.**

### Evaluation du caractère collant de gels aqueux contenant de la glycérine

On prépare un gel aqueux en mélangeant 1,875g du Polyélectrolyte 3, 25g de glycérine et 223,125g d'eau permutée.

A titre de base de comparaison on prépare un gel aqueux en mélangeant 4,7g de SEPIGEL™ 305, 25g de glycérine et 220,3g d'eau.

En étalant chacun des deux gels sur une surface différente du dessus de la main, on observe l'absence d'effet collant pour le gel comprenant le Polyélectrolyte 3 selon l'invention contrairement au gel selon l'état de la technique.

### Exemple 4 : Préparation d'un copolymère AA/ACR D2/MAS

On opère de la même façon qu'à l'exemple 3 mais en ajoutant en plus 8,1g de méthacrylate de stéaryle. On obtient le Polyélectrolyte 4.

### Evaluation du pouvoir épaississant

Viscosité (µ) d'une dispersion aqueuse à 0,5% massique de Polyélectrolyte 4, le pH étant ajusté à 7 [Brookfield RVT, (M6, V5)] : **µ = 15.600 mPas.**

Viscosité (µ) d'une dispersion aqueuse à 1% massique de Polyélectrolyte 4 le pH étant ajusté à 7 et 1‰ massique de chlorure de sodium [Brookfield RVT, (M6, V5)] : **µ = 13.100 mPas.**

### Evaluation du caractère collant de gels aqueux contenant de la glycérine

On prépare un gel aqueux en mélangeant 1,875g du Polyélectrolyte 4, 25g de glycérine et 223,125g d'eau permutée.

A titre de base de comparaison on prépare un gel aqueux en mélangeant 4,7g de SEPIGEL™ 305, 25g de glycérine et 220,3g d'eau.

En étalant chacun des deux gels sur une surface différente du dessus de la main, on observe l'absence d'effet collant pour le gel comprenant le Polyélectrolyte 4 selon l'invention contrairement au gel selon l'état de la technique.

### Exemples de formulations comprenant de la glycérine et exemptes d'huiles de silicone préparées avec des polyélectrolytes selon l'invention

### Exemple 5 : Emulsion démaquillante à l'huile d'amandes douces

| | |
|---|---|
| MONTANOV™ 68 : | 5% |
| Huile d'amandes douces : | 5% |
| Eau : | q.s.p. 100% |
| Polyélectrolyte 1 : | 0,3% |
| Glycérine : | 5% |
| Conservateur : | 0,2% |
| Parfum : | 0,3% |

### Exemple 6 : Emulsion pour peaux à tendance atopique

| | |
|---|---|
| ARLACEL™ P135 : | 2,00% |
| Polyélectrolyte 4: | 1,00% |
| LANOL™ 1688 : | 14,00% |
| PRIMOL™ 352 : | 8,00% |
| Glycérine : | 5,00% |
| Eau: | qsp 100% |
| Sulfate de magnésium : | 0,70% |
| SEPICIDE™ HB : | 0,30% |
| SEPICIDE™ Cl : | 0,20% |
| MICROPEARL™ M310 : | 5,00% |

Les définitions des produits commerciaux utilisés dans les exemples, sont les suivantes :
Le MONTANOV™ 68 (cétéaryl glucoside), est une composition auto-émulsionnable telle que décrite dans WO 92/06778, commercialisée par la société SEPPIC.
L'ARLACEL™ P135 est un mélange de monostéarate de glycérol, de distéarate de glycérol et de polyoxyéthylène-stéarate de glycérol commercialisé par la société CRODA.
Le SEPICIDE™ CI, imidazolidine urée, est un agent conservateur commercialisé par la société SEPPIC.
Le LANOL™ 1688 est un ester émollient à effet non gras commercialisé par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
Le MICROPEARL™ M 310 est une poudre ultra fine au toucher très doux et à action matifiante commercialisée par la société MATSUMO.
Le PRIMOL™ 352 est une huile minérale commercialisée par la société EXXON.

## Revendications

1. Polyélectrolyte anionique linéaire, branché ou réticulé, issu de la polymérisation pour 100% massique :
a) d'une proportion massique supérieure ou égale à 80% et inférieure ou égale à 99,5% d'unités monomériques issues d'un monomère comportant une fonction acide faible ;
b) d'une proportion massique supérieure ou égale à 0,5% et inférieure ou égale à 20%,
- Soit d'un monomère de formule (Ia) :
R-(CH₂)₃-Si(CH₃)₂-[O-Si(CH₃)₂-]ₙO-Si(CH₃)₂-(CH₂)₃-R (la),
dans laquelle :
R représente le radical monovalent :
-(O-CH₂-CH₂-)ₓ[O-CH₂-CH(CH₃)-]_{y}O-C(=O)-CH=CH₂,
dans lequel x et y représentent, indépendant l'un de l'autre, un nombre entier supérieur ou égal à 0 et inférieur ou égal à 40, étant entendu que la somme x + y est supérieure à 0 et inférieure à 50 ; et
n représente un nombre entier supérieur ou égal à 0 et inférieur ou égal à 45, ledit monomères de formule (la) ayant un poids moléculaire supérieur ou égal à 1.500 et inférieur ou égal à 7.000 ;
- Soit d'un monomère de formule (Ib) :
Si(CH₃)₃-[O-Si(CH₃)₂-]ₘ[O-Si(CH₃)[(CH₂)₃-R]-]ₚO-Si(CH₂)₃ (Ib),
dans laquelle :
R représente un radical monovalent de formule :
-(O-CH₂-CH₂-)ₓ[O-CH₂-CH(CH₃)-]_{y}O-C(=O)-CH=CH₂,
dans lequel x et y représentent, indépendant l'un de l'autre, un nombre entier supérieur ou égal à 0 et inférieur ou égal à 40, étant entendu que la somme x + y est supérieure à 0 et inférieure à 50 ;
m représente un nombre entier supérieur ou égal à 1 et inférieur ou égal à 7, et
p représente indépendamment de m un nombre entier supérieur ou égal à 1 et inférieur à 20 ;
ledit monomères de formule (Ib) ayant un poids moléculaire supérieur ou égal à 1.000 et inférieur ou égal à 4.000 ;
c) optionnellement d'une proportion massique supérieure à 0% et inférieure ou égale à 5%, d'unités monomériques issues d'au moins un monomère de formule (II) :
R₂-C(=O)-O-[(CH₂-CH(R₄)-O]ₘ-R₃ (II),
formule (II) dans laquelle m représente un nombre supérieur ou égal à 0 et inférieur ou égal à 50, R₂ représente un radical monovalent aliphatique insaturé comprenant de 2 à 4 atomes de carbone, R₄ représente un atome d'hydrogène, un radical méthyle ou un radical éthyle et R₃ représente un radical aliphatique hydrocarboné, linéaire ou ramifié, saturé ou insaturé, comprenant de 8 à 30 atomes de carbone, et
d) optionnellement d'une proportion massique supérieure à 0% et inférieure ou égale à 5% d'au moins un monomère de réticulation diéthylénique ou polyéthylénique.

2. Polyélectrolyte anionique tel que défini à la revendication 1, pour lequel ledit monomère comportant une fonction acide faible est choisi parmi l'acide acrylique, l'acide méthacrylique, l'acide itaconique, l'acide maléique ou l'acide 3-méthyl 3-[(1-oxo 2-propènyl) amino] butanoïque.

3. Polyélectrolyte anionique tel que défini à la revendication 2, pour lequel ledit monomère comportant une fonction acide faible est l'acide acrylique.

4. Polyélectrolyte anionique tel que défini à l'une quelconque des revendications 1 à 3, pour lequel, ledit monomère de formule (II), est choisi parmi le méthacrylate de béhènyle pentacosaéthoxylé, l'acrylate de lauryle tétraéthoxylé, le méthacrylate de stéaryle eicosaéthoxylé, le méthacrylate de lauryle tétraéthoxylé, ou le méthacrylate de stéaryle.

5. Polyélectrolyte anionique tel que défini à l'une quelconque des revendications 1 à 4, issu de la polymérisation pour 100% massique :
a) d'une proportion massique supérieure ou égale à 85% et inférieure ou égale à 98% d'unités monomériques issues d'un monomère comportant une fonction acide faible,
b) d'une proportion massique supérieure ou égale à 1% et inférieure ou égale à 14% d'unités monomériques issues d'un monomère de formule (la) ou d'un monomère de formule (Ib) telles que définies précédemment ; et
c) d'une proportion massique supérieure ou égale à 1% et inférieure ou égale à 5% d'unités monomériques issues du composé de formule (II) telle que définie précédemment.

6. Polyélectrolyte anionique tel que défini à l'une quelconque des revendications 1 à 5, **caractérisé en ce qu'**il est réticulé.

7. Polyélectrolyte anionique tel que défini à la revendication 6, **caractérisé en ce que** ledit au moins un monomère de réticulation diéthylénique ou poly-éthylénique est choisi parmi le méthylène bis(acrylamide) ou le propanetriacrylate de triméthylol propane.

8. Procédé de préparation du polyélectrolyte tel que défini à l'une quelconque des revendications 1 à 7, **caractérisé en ce qu'**il comprend:
Une étape a) de préparation d'un mélange réactionnel comprenant dans les proportions molaires souhaitées et dans un solvant organique (S), le ou les monomères comportant une fonction acide faible, le monomère de formule (la) ou le monomère de formule (Ib) ; si nécessaire ou si désiré les unités monomériques issues du composé de formule (II) ; et si nécessaire ou si désiré le ou les monomères de réticulation diéthyléniques ou polyéthyléniques,
Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte ; si nécessaire ou si désiré :
une étape c) d'isolation dudit précipité obtenu à l'étape b) par séparation d'avec ledit solvant (S), puis si nécessaire ou si désiré,
une étape d) de séchage dudit précipité résultant de l'étape c).

9. Procédé tel que défini à la revendication 8, dans lequel le solvant (S) est un mélange cyclohexane-acétate d'éthyle.

10. Utilisation du polyélectrolyte anionique tel que défini à l'une quelconque des revendications 1 à 7, comme épaississant et/ou comme stabilisant et/ou comme émulsionnant, d'une composition topique cosmétique, dermopharmaceutique ou pharmaceutique.

11. Composition topique cosmétique **caractérisée en ce qu'**elle comprend de 1% à 5% massique du polyélectrolyte anionique tel que défini à l'une quelconque des revendications 1 à 7, et de 1% massique à 10% massique de glycérol.

12. Composition telle que définie à la revendication 11, **caractérisée en ce qu'**elle est exempte d'huile de silicone.

## Patentansprüche

1. Verzweigter oder vernetzter linearer anionischer Polyelektrolyt aus der Polymerisation für 100 Gew**.-**%**:**
a) eines Gewichtsverhältnisses größer oder gleich 80% und kleiner oder gleich 99,5% an Monomereinheiten aus einem Monomer, eine schwache Säurefunktion umfassend;
b) eines Gewichtsverhältnisses größer oder gleich 0,5% und kleiner oder gleich 20%,
- oder eines Monomers der Formel (Ia):
R- (CH₂)₃-Si (CH₃)₂-[O-Si (CH₃)₂- ]ₙO-Si (CH₃)₂- (CH₂)₃-R (Ia)
wobei:
R das einwertige Radikal darstellt:
- (O-CH₂-CH₂-)ₓ[O-CH₂-CH(CH₃)-]_{y}O-C(=O)-CH=CH₂,
wobei x und y unabhängig voneinander eine ganze Zahl größer oder gleich 0, und kleiner oder gleich 40 darstellen, vorausgesetzt, dass die Summe von x + y größer als 0 ist und kleiner als 50 ist; und
n eine ganze Zahl größer oder gleich 0, und kleiner oder gleich 45 darstellt, wobei das besagte Monomer der Formel (Ia) ein Molekulargewicht größer oder gleich 1.500 und kleiner oder gleich 7.000 aufweist;
- oder eines Monomers der Formel (Ib):
Si (CH₃)₃- [O-Si (CH₃)2-]ₘ [O-Si (CH₃) [(CH₂)₃-R] -]ₚO-Si (CH₂)₃ (Ib)
wobei:
R ein einwertiges Radikal der folgenden Formel ist:
- (O-CH₂-CH₂-)ₓ[O-CH₂-CH(CH₃)-]_{y}O-C(=O)-CH=CH₂,
wobei x und y unabhängig voneinander eine ganze Zahl größer oder gleich 0, und kleiner oder gleich 40 darstellen, vorausgesetzt, dass die Summe von x + y größer als 0 ist und kleiner als 50 ist;
m eine ganze Zahl größer oder gleich 1 und kleiner oder gleich 7 ist, und
p unabhängig von m ganze Zahl größer oder gleich 1 und kleiner 20 ist;
wobei das besagte Monomer der Formel (Ib) ein Molekulargewicht größer oder gleich 1.000 und kleiner oder gleich 4.000 aufweist;
c) optional eines Gewichtsverhältnisses größer als 0% und kleiner oder gleich 5%, aus Monomereinheiten aus zumindest einem Monomer der Formel (II):
R₂-C(=O)-O-[(CH₂-CH(R₄)-O]ₘ-R₃ (II)
Formel (II), in der m eine Zahl größer oder gleich 0, und kleiner oder gleich 50 ist, R₂ ein nicht saturiertes aliphatisches einwertiges Radikal mit 2 bis 4 Kohlenstoffatomen ist, R₄ ein Wasserstoffatom, ein Methylradikal oder ein Ethylradikal darstellt, und R₃ ein lineares oder verzweigtes, saturiertes oder nicht saturiertes aliphatisches Kohlenwasserstoffradikal mit 8 bis 30 Kohlenstoffatomen ist, und
d) optional eines Gewichtsverhältnisses größer als 0% und kleiner oder gleich 5%, aus zumindest einem diethylenischen oder polyethylenischen Vernetzungsmonomer.

2. Anionischer Polyelektrolyt nach Anspruch 1, wobei das besagte Monomer eine schwache Säurefunktion umfassend aus Acrylsäure, Metacrylsäure, Itaconsäure, Maleinsäure oder 3-Methyl 3-[(1-oxo 2-Propenyl) Amino] Buttersäure ausgewählt wird.

3. Anionischer Polyelektrolyt nach Anspruch 2, wobei das besagte Monomer eine schwache Säurefunktion umfassend Acrylsäure ist.

4. Anionischer Polyelektrolyt nach einem der Ansprüche 1 bis 3, wobei das besagte Monomer der Formel (II) aus pentacosaethoxyliertem Behenylmetacrylat, tetraethoxyliertem Laurylmetacrylat oder Stearylmetacrylat ausgewählt wird.

5. Anionischer Polyelektrolyt nach einem der Ansprüche 1 bis 4, aus der Polymerisation für 100 Gew**.-**%**:**
a) eines Gewichtsverhältnisses größer oder gleich 85% und kleiner oder gleich 98% an Monomereinheiten aus einem Monomer, eine schwache Säurefunktion umfassend,
b) eines Gewichtsverhältnisses größer oder gleich 1% und kleiner oder gleich 14% an Monomereinheiten aus einem Monomer der Formel (Ia) oder aus einem Monomer der Formel (Ib), wie zuvor definiert; und
c) eines Gewichtsverhältnisses größer oder gleich 1% und kleiner oder gleich 5% an Monomereinheiten aus der Zusammensetzung der Formel (II), wie zuvor definiert.

6. Anionischer Polyelektrolyt nach einem der Ansprüche 1 bis 5, **dadurch gekennzeichnet, dass** er vernetzt ist.

7. Anionischer Polyelektrolyt nach Anspruch 6, **dadurch gekennzeichnet, dass** das zumindest ein diethylenisches oder polyethylenisches Vernetzungsmonomer aus Methylenbisacrylamid oder Trimethylolpropantriacrylat ausgewählt wird.

8. Verfahren zur Herstellung des Polyelektrolyts nach einem der Ansprüche 1 bis 7, **dadurch gekennzeichnet, dass** es Folgendes umfasst:
einen Schritt a) zur Herstellung einer Reaktionsmischung, die in den gewünschten Molverhältnissen und in einem organischen Lösungsmittel (S) das oder die Monomere mit einer schwachen Säurefunktion, das Monomer der Formel (Ia) oder das Monomer der Formel (Ib) umfasst; wenn nötig oder wenn gewünscht die Monomereinheiten aus der Zusammensetzung der Formel (II); und wenn nötig oder wenn gewünscht das oder die diethylenischen oder polyethylenischen Vernetzungsmonomere,
einen Schritt b) im Laufe dessen die Polymerisationsreaktion durch Einfügen eines Initiators freier Radikale in die besagte Reaktionsmischung, die in Schritt a) hergestellt wurde, ausgelöst wird, die man danach bis zum Abschluss ablaufen lässt, um eine Ausfällung des besagten Polyelektrolyts zu erhalten;
wenn nötig oder wenn gewünscht:
einen Schritt c) zum Isolieren der besagten im Schritt b) erhaltenen Ausfällung durch die Trennung mit dem besagten Lösungsmittel (S), danach wenn nötig oder wenn gewünscht,
einen Schritt d) zum Trocknen der besagten sich aus dem Schritt c) ergebenden Ausfällung.

9. Verfahren nach Anspruch 8, wobei das Lösungsmittel (S) eine Ethyl-Cyclohexanacetat Mischung ist.

10. Verwendung eines anionischen Polyelektrolyts nach einem der Ansprüche 1 bis 7 als Verdickungsmittel und/ oder als Stabilisator und/ oder als Emulsionsmittel einer topischen kosmetischen, hautkosmetischen oder pharmazeutischen Zusammensetzung.

11. Topische kosmetische Zusammensetzung, **dadurch gekennzeichnet, dass** sie 1 Gew**.-**% bis 5 Gew**.-**% an anionischem Polyelektrolyt nach einem der Ansprüche 1 bis 7 umfasst, und 1 Gew**.-**% bis 10 Gew**.-**% an Glycerin.

12. Zusammensetzung nach Anspruch 11, **dadurch gekennzeichnet, dass** sie frei von Silikonöl ist.

## Claims

1. Linear, branched or cross-linked anionic polyelectrolyte, resulting from the polymerisation for 100% by mass:
a) of a mass ratio greater than or equal to 80% and less than or equal to 99.5% of monomeric units from a monomer comprising a weak acid function;
b) of a mass ratio greater than or equal to 0.5% and less than or equal to 20%,
- Either of a monomer of formula (Ia):
R- (CH₂)₃-Si (CH₃)₂-[O-Si (CH₃)₂-]ₙO-Si (CH₃)₂(CH₂)₃-R (Ia),
wherein:
R represents the monovalent radical:
- (O-CH₂-CH₂-)ₓ[O-CH₂-CH(CH₃)-]_{y}O-C(=O) -CH=CH₂,
wherein x and y represent, independently from one another, an integer greater than or equal to 0 and less than or equal to 40, with the understanding that the sum x + y is greater than 0 and less than 50; and
n represents an integer greater than or equal to 0 and less than or equal to 45, said monomers of formula (Ia) having a molecular weight greater than or equal to 1,500 and less than or equal to 7,000;
- Or of a monomer of formula (Ib):
Si (CH₃)₃-[O-Si (CH₃)₂-]ₘ[O-Si (CH₃)[(CH₂)₃-R] - ]ₚO-Si (CH₂)₃ (Ib),
wherein:
R represents a monovalent radical of formula:
- (O-CH₂-CH₂-)ₓ[O-CH₂CH(CH₃)-]_{y}O-C(=O)-CH=CH₂,
wherein x and y represent, independently from one another, an integer greater than or equal to 0 and less than or equal to 40, with the understanding that the sum x + y is greater than 0 and less than 50;
m represents an integer greater than or equal to 1 and less than or equal to 7, and
p represents independently of m an integer greater than or equal to 1 and less than 20;
said monomers of formula (Ib) having a molecular weight greater than or equal to 1,000 and less than or equal to 4,000;
c) optionally of a mass ratio greater than 0% and less than or equal to 5%, of monomeric units from at least one monomer of formula (II):
R₂-C(=0)-0-[(CH₂CH(R₄)-O]ₘ-R₃ (II),
formula (II) wherein m represents a number greater than or equal to 0 and less than or equal to 50, R₂ represents an unsaturated aliphatic monovalent radical comprising from 2 to 4 carbon atoms, R₄ represents a hydrogen atom, a methyl radical or an ethyl radical and R₃ represents a saturated or unsaturated, linear or branched hydrocarbon aliphatic radical, comprising from 8 to 30 carbon atoms, and
d) optionally of a mass ratio greater than 0% and less than or equal to 5% of at least one monomer with diethylenic or polyethylenic cross-linking.

2. Anionic polyelectrolyte such as defined in claim 1, for which said monomer comprising a weak acid function is chosen from acrylic acid, methacrylic acid, itaconic acid, maleic acid or 3-methyl 3-[(1-oxo 2-propenyl) amino] butanoic acid.

3. Anionic polyelectrolyte such as defined in claim 2, for which said monomer comprising a weak acid function is acrylic acid.

4. Anionic polyelectrolyte such as defined in any of claims 1 to 3, for which, said monomer of formula (II), is chosen from pentacosaethoxylated behenyl methacrylate, tetraethoxylated lauryl acrylate, eicosaethoxylated stearyl methacrylate, tetraethoxylated lauryl methacrylate, or stearyl methacrylate.

5. Anionic polyelectrolyte such as defined in any of claims 1 to 4, from the polymerisation for 100% by mass:
a) of a mass ratio greater than or equal to 85% and less than or equal to 98% of monomeric units from a monomer comprising a weak acid function,
b) of a mass ratio greater than or equal to 1% and less than or equal to 14% of monomeric units from a monomer of formula (Ia) or from a monomer of formula (Ib) such as defined hereinabove; and
c) of a mass ratio greater than or equal to 1% and less than or equal to 5% of monomeric units from the compound of formula (II) such as defined hereinabove.

6. Anionic polyelectrolyte such as defined in any of claims 1 to 5, **characterised in that** it is cross-linked.

7. Anionic polyelectrolyte such as defined in claim 6, **characterised in that** said at least one monomer with diethylenic or polyethylenic cross-linking is chosen from methylene bisacrylamide or trimethylol propane propanetriacrylate.

8. Method for preparing polyelectrolyte such as defined in any of claims 1 to 7, **characterised in that** it comprises:
A step a) of preparing a reaction mixture comprising in the desired molar proportions and in an organic solvent (S), the monomer or monomers comprising a weak acid function, the monomer of formula (Ia) or the monomer of formula (Ib); if necessary or if desired the monomeric units from the compound of formula (II); and if necessary or if desired the monomer or monomers with diethylenic or polyethylenic cross-linking,
A step b) during which the polymerisation reaction is primed by the introduction into said reaction mixture prepared in step a), of a free radical initiator, then is allowed to unfold until the conclusion thereof, in order to obtain a precipitate of said polyelectrolyte; if necessary or if desired:
a step c) of isolating said precipitate obtained in the step b) by separation with said solvent (S), then if necessary or if desired,
a step d) of drying said precipitate resulting from the step c).

9. Method such as defined in claim 8, wherein the solvent (S) is a cyclohexane-ethyl acetate mixture.

10. Use of anionic polyelectrolyte such as defined in any of claims 1 to 7, as a thickener and/or as a stabiliser and/or as an emulsifier, of a topical cosmetic, dermopharmaceutical or pharmaceutical composition.

11. Topical cosmetic composition **characterised in that** it comprises from 1% to 5% by mass of the anionic polyelectrolyte such as defined in any of claims 1 to 7, and from 1% by mass to 10% by mass of glycerol.

12. Composition such as defined in claim 11, **characterised in that** it is free from silicone oil.
